(19) Europäisches Patentamt
European Patent Office
Office européen des brevets

(11) Publication number: **0 189 101 A2**

(12) **EUROPEAN PATENT APPLICATION**

(21) Application number: 86100442.2

(22) Date of filing: 15.01.86

(51) Int. Cl.[4]: C 07 D 473/08, A 61 K 31/52

(30) Priority: 23.01.85 IT 1920485

(43) Date of publication of application: 30.07.86 Bulletin 86/31

(84) Designated Contracting States: BE CH DE FR GB IT LI

(71) Applicant: PROTER S.p.A., 38 Via Lambro, I-20090 Opera (Milan) (IT)

(72) Inventor: Dall'Asta, Leone, Viale Damiano Chiesa 26, I-27100 Pavia (IT)
Inventor: Coppi, Germano, Via Morandi 5, I-20094 Buccinasco Milan (IT)
Inventor: Springolo, Vanna, Via Vittadini 3, I-20136 Milan (IT)
Inventor: Scevola, Mario Ercole, Via Tuberose 4, I-20146 Milan (IT)

(74) Representative: Dragotti, Gianfranco et al, SAIC BREVETTI s.a.s. Viale Bianca Maria, 15, I-20122 Milano (IT)

(54) **Compounds having mucolytic and bronchodilating activity, a process for their preparation and pharmaceutical composition containing them as active principle.**

(57) Novel compounds having mucolytic and bronchodilating activity, a process for their preparation consisting in the treatment of 7-hydroxymethyl-theophylline with succinis anhydride to give 7-succinoyloxymethyl-theophylline, which in turn is converted in an activated derivative to carry out the condensation reaction with the corresponding thiol; pharmaceutical formulations containing said compounds as active ingredients.

EP 0 189 101 A2

0189101

Specification of the industrial patent application having the title: "Compounds having mucolytic and bronchodilating activity, a process for their preparation and pharmaceutical compositions containing them as active principle"

in the name of Proter Spa, having head office at Opera (Mi)

Inventors: Leone Dall'Asta, Germano Coppi, Vanna Springolo and Mario Ercole Scevola

filed on 23.01.1985 to the No. 19204 A/85

ABSTRACT

Novel compounds having mucolytic and bronchodilating activity;a process for their preparation consisting in the treatment of 7-hydroxymethyl--theophylline with succinic anhydride to give 7-succinoyloxymethyl)-theophylline, which in turn is converted in an activated derivative to carry out the condensation reaction with the corresponding thiol; pharmaceutical formulations containing said compounds as active ingredient.

SPECIFICATION

The present invention relates, according to one feature, to compounds having the formula I:

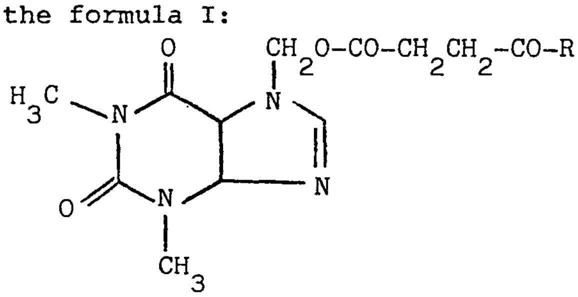

(I)

wherein R represents the radicals:- 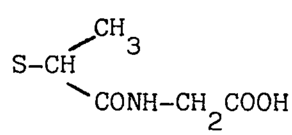

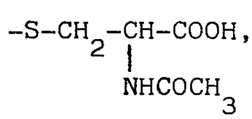, 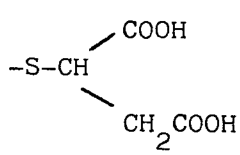, $-S-CH_2CH_2-SO_3H$ or 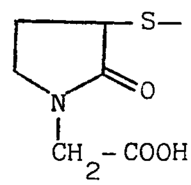

According to a further feature, the present invention relates to a process for the preparation of the compounds of formula I and possibly of

the salts with inorganic or organic bases, characterized in that 7-hydroxymethyl-theophylline of formula II:

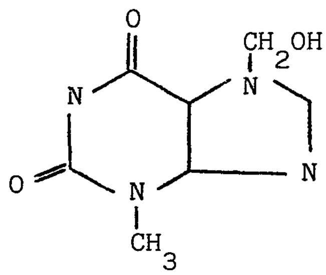

(II)

is treated with succinic anhydride to give the compound III:

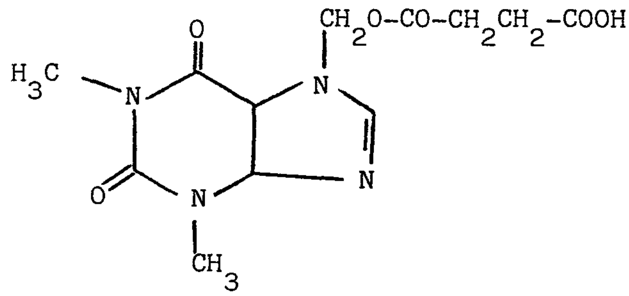

(III)

Subsequently the compound of formula III, in activated form, is reacted with the thiol derivative to give the compound of formula I.

The thiol compounds used are known in the literature, particularly the 3-mercapto-2-oxo-pyrrolidinacetic acid was claimed by the applicant in the Italian Patent Application No. 20315 A/84.

The compound of formula II is prepared as described by K.B. Sloan and N.Bodor, Int. J.Pharm. 12, 299 (1982).

The preferred way for the preparation of compound of formula I may be exemplified as follows:

In the first phase the 7-hydroxymethyl-theophylline (II) is reacted with succinic anhydride ; the reaction takes place in a suitable solvent, such as methylene chloride, in the presence of an organic base, for instance triethylamine. The amount of succinic anhydride is in excess with respect to the calculated one; such an excess may vary between 50% and 200%, the stoichiometrical amount being preferably doubled. Thus the amount of organic base used is preferably two times with respect to the moles of compound II. The reaction mixture is stirred for a time period variable between 10 minutes and 3 hours, preferably for 30 minutes at a temperature of between 0° and 50°C, preferably at 20-25°C.

The isolation of the compound of formula III takes place by dilution of the reaction mixture with an aqueous solution of sodium bicarbonate,so as to solubilize the compound III in form of sodium salt, and by subsequent acidification of the aqueous solution through the addition of diluted mineral acid, preferably 6N hydrochloric acid.

In the subsequent phase the compound III is dissolved in a suitable solvent, such as dioxane, by adding an organic base, preferably tributylamine; the salification reaction takes place at temperatures of between 20° and 100°C, preferably at 45-50°C for times between of 10 minutes and 3 hours, preferably 30 minutes.

The subsequent forming of the mixed anhydride takes place through the addition to the reaction mixture of an alkyl chlorocarbonate, preferably ethylchlorocarbonate, at temperatures between -10°C and +30°C, preferably at 5-10°C, for reaction times of between 10 minutes and 3 hours, preferably 40 minutes.

The condensation reaction to give the compound of formula I, wherein for example R is

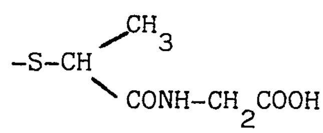

is carried out by adding to the solution of the mixed anhyride a solution of 2-mercapto-propionylglycine in form of the sodium salt in an aqueous solvent, preferably 30% dioxane. The reaction time is of between 1 hour and 12 hours, preferably 6 hours, at temperatures of between 0°C and 50°C, preferably at 20-25°C. The isolation of the final product takes place by evaporation of the resulting solution under reduced pressure, by subsequent solubilization of the residue oil through the addition of an aqueous solution of sodium bicarbonate, by colouring and precipitating again the compound I by acidification at pH 2 through the addition of mineral acid, preferably 6N hydrochloric acid. The operations of solubilizing and then again precipitating the compound I are carried out at temperatures of between 0 and 25°C, preferably at 10-15°C.

The compounds of formula I possess interesting pharmacological properties; particularly they are endowed with a remarkable mucolytic, expectorant anticough, antibronchospastic, bronchodilating activity and moreover show low toxicity. More specifically in the test of "in vitro" mucolytic activity with a suspension of 4% mucine (II Farmaco, ed.prat.33-379, 1978), and the molar concentration being the same (0,00625 M), after hydrolysis with rat serum, the 2-/(theophyllin-7-yl)-methyloxysuccinoylthio7propionamido acetic acid caused a reduction of viscosity by 11,2% to take place, which is like that of tiopronin (11,5%) and higher than that of N-acetylcysteine (7,6%). Moreover the compounds of the present invention possess a remarkable mucolytic-expectorant activity, shown in the test of fluorescein elimination in the fluid of the respiratory tract (II Farmaco, ed. prat. 36, 167, 1981), in which test, a representative compound, 2-/(theophyllin-7-yl)-methyloxisuccinoylthio7-propionamido acetic acid, demonstrated in comparison with the controls, an increase of the escretion of the colouring compound by 86%, which is not significatively different from that of tiopronin (94%), but significatively higher than that of N-acetylcysteine at the same dosages (1 mM/kg/os) and under the same experimental conditions.

The anticough activity of the compounds of the present invention has been evaluated in the test of the cough induced by citric acid in the guinea pig (Screening Methods in Pharmacology, R.A. Turner, Acad. Press 1965, 219) wherein a representative compound, 2-/(theophyllin-7-yl)-methyloxysuccinoylthio7-propionamido acetic acid, demonstrated in comparison with the controls a reduction of the cough by 79% at the dose of 100 mg/kg/i.p. which is not significatively different from that obtained with codeine phosphate at the dose of 5 mg/kg/i.p. (87%).

The antibronchospastic activity of the compounds of the present invention has been evaluated in the test of the shock induced by histamine in the guinea pig (Screening Methods in Pharmacology, R.A. Turner, Acad. Press 1965, 214) wherein a representative compound, 2-/(theophyllin-7-yl)methoxysuccinoylthio7-propionamido acetic acid, demonstrated an

increase of the shock resistance from 99.8 ± 9.6 seconds to 291.7 ± 43.2 seconds likewise theophylline (from 133.5 ± 24.9 seconds to 316.8 ± 27.3 seconds) at the same dose (0.25 mM/kg/i.p.).

The bronchodilating-antibronchospastic activity of the compounds of the present invention has been evaluated by means of the Konzett-Rössler test (Arch. exp. Path. Pharmakol. 195, 171, 1940) in the guinea pig. A representative compound of formula I, the 2-[(theophyllin-7-yl)-methoxy-succinoylthio]-propionamido acetic acid, demonstrated that it possesses against the spasm induced by acetylcholine (10 mcg/kg/e.v.) an $ED_{50}$ of 0.0623 mM/kg/e.v. (0.0276-0.1406), like that of theophylline of 0.0395 mM/kg/e.v. (0.0149-0.01041) and on the contrary, surprisingly, against the spasm induced by histamine (2 mcg/kg/e.v.), an $ED_{50}$ of 0.0345 mM/kg/e.v. (0.0133-0,893) which is definitely and significatively lower than that of theophylline (0.1026 (0.0508-0.2072). The bronchodilating activity of the compounds of formula I is consequently definitely higher than that of theophylline.

The compounds of the present invention show a low acute toxicity; the representative compound, 2-[(theophyllin-7-yl)-methyloxysuccinoylthio]-propionamido acetic acid, shows in female mouse a $LD_{50}$ value by i.p. route of 481 mg/kg.

Thanks to their pharmacological properties and to the low toxicity, the compounds of formula I and their possible pharmaceutically acceptable salt may be used as drugs in the treatment of diseases of the respiratory tract.

Thus the present invention relates, according to a further feature, to pharmaceutical compositions containing, as the active ingredients, the compounds of formula I or their pharmaceutical acceptable salts for the administration by aerosol, parenteral, oral and rectal route in form of vials, oral suspensions, capsules, tablets, envelopes or suppositories.

Examples of pharmaceutical formulations according to the invention comprise:

- vials containing 50 to 400 mg of active ingredient

- syrup containing 100 to 500 mg of active principles per dose
- capsules of hard gelatine or Scherer type containing 100 to 500 mg of active ingredient
- small envelopes containing 100 to 500 mg of active ingredient in a suitable excipient
- suppositories containing 50 to 600 mg of active ingredient in suitable vehicles for nepiological , pediatric and for adult use.

The daily dosage does vary between 100 and 2000 mg of active ingredient in the treatment of diseases of the respiratory tract, particularly asthma, bronchitis, tracheobronchitis, pharyngitis , etc.

The following examples, having non limiting purposes, illustrate the invention.

EXAMPLE 1

140 g (0.66 moles) of 7-hydroxymethyl-theophylline are suspended in 1400 ml of methylene chloride and under stirring 185.3 ml (1.33 moles) of triethylamine are added. Subsequently 133 g (1.33 moles) of succinic anhydride suspended in 1400 ml of methylene chloride are added; after 10 minutes of stirring a clear solution is obtained, which, after TLC monitoring (eluant: ethylacetate/methanol/acetic acid=80:10:10 v/v/v), is evaporated under reduced pressure at 30-35°C. The residue is taken with a 2.5% (w/v) sodium bicarbonate aqueous solution until a complete solubilization is obtained (about 5 litres of solution). After decolouration and filtration, the solution is slowly added with 6N hydrochloric acid up to pH 4.7; the solid product is filtered, washed with water, dried in air and lastly under vacuum at 40°C.

There are obtained 166 g(81%) of 7-(succinoyloxymethyl)-theophylline, in form of a white product having p.f. 101-102°C (dec.).

Analytical data for $C_{12}H_{14}N_4O_6$ (M.W.: 310.272)

Calculated %= C 46.45 - H 4.55 - N 18.06

Found % = C 46.77 - H 4.48 - N 18.11

The IR (KBr) and $^1$H-NMR (DMSO-$d_6$) spectra are into agreement with the proposed structure.

EXAMPLE 2

2500 ml of dioxane are added with 125 g (0.403 moles) of 7-(succinoyloxymethyl)-theophylline and then under stirring 95.75 ml (0.401 moles) of tributylamine are added.

The reaction mixture is heated to 45-50°C for 30 minutes and a clear solution is obtained; after cooling to + 10°C, 38.25 ml (0.401 moles) of ethylchlorocarbonate, are added keeping the temperature at 5-10°C.

The mixture is maintained under stirring at 5-10°C for 40 minutes, then at the same temperature a solution of 74.5 g (0.402 moles) of sodium salt of 2-mercapto-propionylglicine in 400 ml of water and 185 ml of dioxane is added.Upon the addition is completed, the temperature increases over 6 hours to 20-25°C, then the mixture is maintained at rest for 15 hours at +5°C; the solution is evaporated under reduced pressure until an oil is obtained, which is taken with 200 ml of water and brought to pH 7.5-8 through the addition of a 5% (w/v) aqueous solution of sodium bicarbonate. The opalescent solution is added with decolouring carbon, filtered and made acidic at pH 2 through the addition of 6N hydrochloric acid, the temperature being maintained at 10-15°C. The mixture is cooled to +5°C and stirred for 15 minutes, then maintained at 5°C for 18 hours.

The white solid product is filtered and washed with cold water until the chlorides disappear. It is dried in air, then under vacuum at 30°C in the presence of phosphoric anhydride.

111 g (61%) of 2-[(theophyllin-7-yl)methyloxysuccinoylthio]-propionamido acetic acid are obtained, in form of a crystalline product having p.f. 115-123°C (dec.).

Analytical data for $C_{17}H_{21}N_5O_8S$ (M.W.: 455.454).

Calculated %= C 44.83 - H 4.65 - N 15.38 - S 7.04

Found % = C 44.47 - H 4.72 - N 15.41 - S 7.11

The IR (KBr) and $^1$H-NMR (DMSO-$d_6$) spectra are into agreement with foreseen structure.

To a solution of 9.1 g (0.02 moles) of the thus obtained product in 45 ml of ethyl acetate 20 ml of a 1 m solution of ethyl hexanoate in ethyl acetate are added. After 30 minutes stirring, the mixture is cooled to 5°C, filtered, washed with ethyl acetate and dried under vacuum at 35°C. There are obtained 8.95 g (93%) of sodium 2-[(theophyllin-7-yl)methyloxy-succinoylthio]-propionamido acetate in white crystalline form.

CLAIMS

1. A compound of formula:

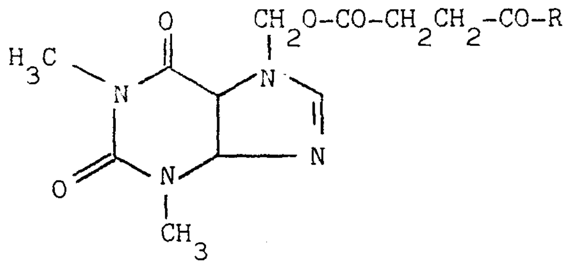

(I)

wherein R is a group: 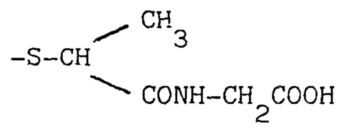

or pharmaceutically acceptable salts of this compound.

2. A compound according to claim 1 having the formula I, wherein R is a group: 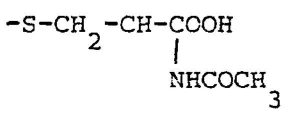

or pharmaceutically acceptable salts thereof.

3. A compound according to claim 1 having the formula I, wherein R is a group:

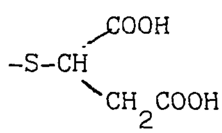

or pharmaceutically acceptable salts thereof.

4. A compound according to claim 1 having the formula I, wherein R is a group: $-S-CH_2CH_2-SO_3H$ or pharmaceutically acceptable salts thereof.

5. A compound according to claim 1 having the formula I, wherein R is a group:

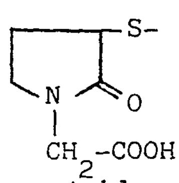

or pharmaceutically acceptable salts thereof.

6. 2-/(theophyllin-7-yl)methyloxisuccinoylthio/-propionamido acetic acid or a pharmaceutically acceptable salt thereof.

7. Sodium 2-/(theophyllin-7-yl)methyloxysuccinoylthio/-propionamido acetate.

8. A process for the preparation of a compound according to claim 1,

characterized in that an activated derivative of 7-(succinoyloxymethyl)-theophilline of formula II is reacted with a thiol.

9. A process for the preparation of a compound according to claim 8, wherein the activated compound of 7-(succinoiloxymethyl)-theophylline is mixed anhydride.

10. Pharmaceutical composition containing, as the active ingredient, a compound according to one of the claims 1-5.

11. Pharmaceutical composition according to claim 10, characterized in that it is formulated in dosing units.

12. Pharmaceutical composition according to claim 11, containing 50-600 mg of active ingredient per dosing unit.

13. Pharmaceutical composition according to claim 12, containing 50-600 mg of active ingredient per dosing unit, in admixture with a pharmaceutically excipient.